# EUROPEAN PATENT APPLICATION

(11) **EP 1 127 559 A1**
(43) Date of publication of application: **29.08.2001**
(21) Application number: 00200566.8
(22) Date of filing: 18.02.2000
(51) Int. Cl.: A61F 2/30

(54) **Plug for insertion into a bone canal**

(71) Applicant: IsoTis N.V., 3723 MB Bilthoven (NL)
(72) Inventor: The designation of the inventor has not yet been filed
(74) Representative: Ottevangers, Sietse Ulbe

(57) **Abstract**

Plug (1) for insertion into a bone canal (8). The plug (1) comprises a central body (2) having fixing means (3) for fixing engagement of the walls (10) of the bone canal (8). The plug (1) further comprises blocking means (4) for blocking cement (22) passage of the bone canal (8) by sealing engagement of the walls (1c) of the bone canal (8). The fixing means (3) and the blocking means (4) extend radially outward relative to a central axis (A) of the plug (1). The blocking means (4) are axially spaced from said central body (2) and are swivably (5) connected thereto. Upon insertion of the plug (1) into a bone canal (8) the blocking means (4) can swivel relative to the central body (2) to sealingly engage a peripheral portion (6) of the blocking means (4) with the walls (10) of the bone canal (8).

## Description

The invention relates to a plug for insertion in a bone canal.

Plugs for insertion into a bone canal are generally known and are used to restrict the flow of cement during prosthesis implantation.

In implant surgery and in particular for implantation of a hip prosthesis, it is common practise to cement the prosthesis to the bone. In order to cement the prosthesis to the bone, the bone canal is broached or reamed, such that the trabecular, porous bone portions are removed and the remaining cortical, hard bone portions define the walls of the bone canal. An anchoring portion of the prosthesis is then inserted into the bone canal, for example an anchoring portion of a hip prothesis is inserted into a broached femural bone, such that a ball portion of the prothesis extends outward to replace the natural ball portion of the hip bone.

In order to fix the anchoring portion of the prosthesis to the bone, cement, such as polymethylmethacrylate (PMMA) is inserted into the bone canal and is packed upon the prosthesis prior to insertion thereof. This cement then anchors the anchoring portion of the prosthesis to the bone material.

In order to provide for a secure joint between the prosthesis and the bone at the cement interface, it is desired to have the cement completely surround the prosthesis in the interstices between the prosthesis and the bone material. However, the insertion forces and pressures exerted during insertion of the implant often drive the cement substance down into the intramedullary canal and away from the fixation area. This way, voids are formed in the cement mantle which later become stress points leading to early fatigue of the prothesis and/or the fixation.

In order to restrict the flow of bone cement further into the intramedullary canal, intramedullary plugging devices or "plugs" have been developed which can be inserted into the bone canal as a blockage. Usually, the plug is provided with fins or other flange-like projections. These fins or flanges serve for simultaneous fixing of the plug in the bone canal by clamping engagement of the walls and for blocking cement passage of the bone canal. When the implant is forced into the broached portion of the intramedullary canal, the tendency of the restricted bone cement to flow down the canal is prevented by the fins or flanges of the plug cooperating with the walls of the canal.

A problem associated with the known plugs is that the fins or flanges do not always cooperate sufficiently sealingly or blockingly with the bone canal. This is largely caused by the fact that the bone canal as defined by the cortical bone has a cross-section that varies along its longitudinal axis. In particular, the cross-section is of substantially elliptical or oval shape at the onset of the canal and axially inwardly becomes more circularly shaped at a midportion. Further inwardly, the cross-section becomes substantially oval or elliptically shaped again, having an orientation that is rotated around the axis of the canal to a position wherein it is substantially perpendicular to the orientation of the first section. In addition, for each person, the size of the bone and therewith the cross-section and axial dimension of the bone canal varies.

During implantation, the plug has to be placed sealingly at a predetermined location within the bone canal, e.g. about 1.5 cm axially inward from the preferred location of the lower tip of the anchoring portion after insertion.

Due to the varying shape of the bone canal it has proven difficult to provide a plug that can be used to reliably plug the canal at any axial location. Depending on the circumstances, a good chance exists that cement can either pass the flanges of the plug or the plug can escape inwardly into the canal upon insertion of the anchoring portion.

The invention aims to provide a plug in which the above problem is overcome. According to the invention, this problem is solved by providing a plug for insertion into a bone canal, comprising a central body having fixing means for fixing the plug in the bone canal by clamping engagement of the walls of the bone canal, further comprising separate blocking means for blocking cement passage of the bone canal by sealing engagement of the walls of the bone canal, said fixing means and said blocking means extending radially outward relative to a central axis of the plug, in which plug the blocking means are axially spaced from said central body and are swivably connected thereto, such that, upon insertion of the plug into the bone canal, the blocking means can swivel relative to the central body to sealingly engage a peripheral portion of the blocking means with the walls of the bone canal.

By providing blocking means that are separate from the fixing means, the fixing means and the blocking means can each be designed to perform its own function. This way, the fixing means can be designed to engage the walls of the bone canal in a primarily fixing fashion, while the blocking means can be designed to engage the walls of the bone canal in a primarily sealing manner. The blocking means can e.g. be designed as relatively stiff flanges or fins, while the blocking means can e.g. be designed as a tiltable flange having a relatively stiff central position and a relatively flexible peripheral portion.

By enabling swivelling movement of the blocking means, the capacity of the blocking means to follow the walls of the bone canal with its peripheral portion is greatly increased. In particular, the swivelling movement enables angular adjustment of the blocking means relative to the central body, such that, as the axial distance between a first peripheral portion of the blocking means and the central body is decreased, the axial distance between a second, radially opposite peripheral portion of the blocking means and the central body is increased. The swivelling movement thus allows a first peripheral portion to move closer to the central body while simultaneously allowing a radially opposite, second peripheral portion to move away from the central body and to extend deeper into the bone canal. The swivable connection allows the blocking means to extend obliquely in the bone canal, such that blockage of the canal can be optimized, e.g. when the canal has a substantially elliptical cross-section and the blocking means are substantially cylindrical.

The swivable connection thus enables angular adjustment of the blocking means relative to the central body carrying the fixing means, such that the blocking means can e.g. be angularly adjusted between a position wherein a central axis thereof is aligned, i.e. with the central axis of the plug and/or the fixing means, to a position wherein said axes intersect or cross. When the fixing means and the blocking means are substantially planar, the swivable connection can e.g. enable the blocking means to tilt from a position wherein its plane is substantially parallel to the plane of the fixing means to a position wherein said planes intersect and vice versa.

The swivelling movement of the blocking means also enables the blocking means to pass a more narrow portion of the bone canal, e.g. the ischius of a femur, and, after passing the narrow portion, to swivel back to a blocking position when the passage of the canal increases. The swivelling movement may not only be achieved by insertion into the bone canal, but may also be generated by outward retracting movement of the plug relative to the bone canal.

By providing the blocking means with a substantially cylindrical circumference forming the peripheral portion, the blocking means can be optimized for cooperation with a cylindrical canal having elliptical portions. Preferably, the blocking means are substantially disk-shaped. This way, sealing engagement of the peripheral portion with the walls of the bone canal can be further enhanced.

By using flexible material for the circumferential portion of the blocking means, the blocking means can adapt to the shape of the walls of the bone canal, e.g. to accommodate irregularities. Preferably, the blocking means comprise a relatively stiff, inner portion radially outwardly extending from a central support to a relatively flexible, peripheral portion. This way, the unsupported, stiff inner portion enhances the swivelling movement.

When the radial dimension of the blocking means is chosen substantially equal to or greater than the radial dimension of the fixing means it can be achieved that in most cases, when the fixing means engage the walls of the bone canal, the blocking means engage the walls of the bone canal as well.

In yet another embodiment, the flanges and/or the carrier are made from a biocompatible and/or biodegradable material.

In the context of the present invention, the term biocompatible is intended to refer to materials which may be incorporated into a human or animal body, e.g. in the form of a medical implant, substantially without unacceptable responses of the human or animal. The term biodegradable refers to materials which, after a certain period of time, are broken down in a biological environment. Preferably, the rate of breakdown is chosen similar or identical to the rate at which the body generates autogenous tissue to replace an implant, such as the present plug, of which the biodegradable material is manufactured.

Preferably, at least the peripheral portion of the blocking means and/or the fixing means or, more preferably, the complete plug is made from a swellable material, such that after insertion and upon contact with (body)fluid, the sealing engagement of the blocking means and/or of the walls of the bone canal can be improved further.

A specific class of swellable materials, which has been found to lead to particularly good results, is the class of copolymers of a polyalkylene glycol and an aromatic polyester. Preferably, these copolymers comprise 40-80 wt.%, more preferably 50-70 wt.% of the polyalkylene glycol, and 60-20 wt.%, more preferably 50-30 wt.% of the aromatic polyester. A preferred type of copolymers according to the invention is formed by the group of block copolymers.

Preferably, the polyalkylene glycol has a weight average molecular weight of from 150 to 4000, more preferably of 200 to 1500. The aromatic polyester preferably has a weight average molecular weight of from 200 to 5000, more preferably of from 250 to 4000. The weight average molecular weight of the copolymer preferably lies between 20,000 and 200,000, more preferably between 50,000 and 120,000. The weight average molecular weight may suitably be determined by gel permeation chromatography (GPC). This technique, which is known per se, may for instance be performed using tetrahydrofuran as a solvent and polystyrene as external standard.

In a preferred embodiment, the polyalkylene glycol component has units of the formula -OLO-CO-Q-CO-, wherein O represents oxygen, C represents carbon, L is a divalent organic radical remaining after removal of terminal hydroxyl groups from a poly(oxyalkylene)glycol, and Q is a divalent organic radical.

Preferred polyalkylene glycols are chosen from the group of polyethylene glycol, polypropylene glycol, and polybutylene glycol and copolymers thereof, such as poloxamers. A highly preferred polyalkylene glycol is polyethylene glycol.

The terms alkylene and polyalkylene generally refer to any isomeric structure, i.e. propylene comprises both 1,2-propylene and 1,3-propylene, butylene comprises 1,2-butylene, 1,3-butylene, 2,3-butylene, 1,2-isobutylene, 1,3-isobutylene and 1,4-isobutylene (tetramethylene) and similarly for higher alkylene homologues. The polyalkylene glycol component is preferably terminated with a dicarboxylic acid residue -CO-Q-CO-, if necessary to provide a coupling to the polyester component. Group Q may be an aromatic group having the same definition as R, or may be an aliphatic group such as ethylene, propylene, butylene and the like.

The polyester component preferably has units -O-E-O-CO-R-CO-, wherein O represents oxygen, C represents carbon, E is a substituted or unsubstituted alkylene or oxydialkylene radical having from 2 to 8 carbon atoms, and R is a substituted or unsubstituted divalent aromatic radical.

In a preferred embodiment, the polyester is chosen from the group of polyethylene terephtalate, polypropylene terephtalate, and polybutylene terephtalate. A highly preferred polyester is polybutylene terephtalate.

The preparation of the copolymer will now be explained by way of example for a polyethylene glycol/polybutylene terephtalate copolymer. Based on this description, the skilled person will be able to prepare any desired copolymer within the above described class. An alternative manner for preparing polyalkylene glycol/polyester copolymers is disclosed in US-A-3,908,201.

A polyethylene glycol/polybutylene terephtalate copolymer may be synthesized from a mixture of dimethyl terephtalate, butanediol (in excess), polyethylene glycol, an antioxidant and a catalyst. The mixture is placed in a reaction vessel and heated to about 180°C, and methanol is distilled as transesterification proceeds. During the transesterification, the ester bond with methyl is replaced with an ester bond with butylene. In this step the polyethyene glycol substantially does not react. After transesterification, the temperature is raised slowly to about 245°C, and a vacuum (finally less than 0.1 mbar) is achieved. The excess butanediol is distilled and a prepolymer of butanediol terephtalate condenses with the polyethylene glycol to form a polyethylene/polybutylene terephtalate copolymer. A terephtalate moiety connects the polyethylene glycol units to the polybutylene terephtalate units of the copolymer and thus such copolymer also is sometimes referred to as a polyethylene glycol terephtalate/polybutylene terephtalate copolymer (PEGT/PBT copolymer).

The invention also relates to a method of inserting a plug into a bone canal.

Further preferred embodiments of the invention are described in the appended claims.

The invention will be elucidated further by means of a drawing. In the drawing is:
Fig. 1A a functional representation of the plug according to the invention;
fig. 1B the plug of fig. 1A inserted in a bone canal;
fig. 2A a perspective view of a preferred embodiment of the plug;
fig. 2B a front view of the plug of fig. 2A;
fig. 3 a cross-section of a femur having a reamed bone canal; and
fig. 4 a cross-section of the femur of fig. 3 with the plug in situ and a prosthesis cemented into the bone canal.

The drawings are only schematic representations of exemplary embodiments of the plug. In the drawings, identical or corresponding parts are identified with the same reference numerals.

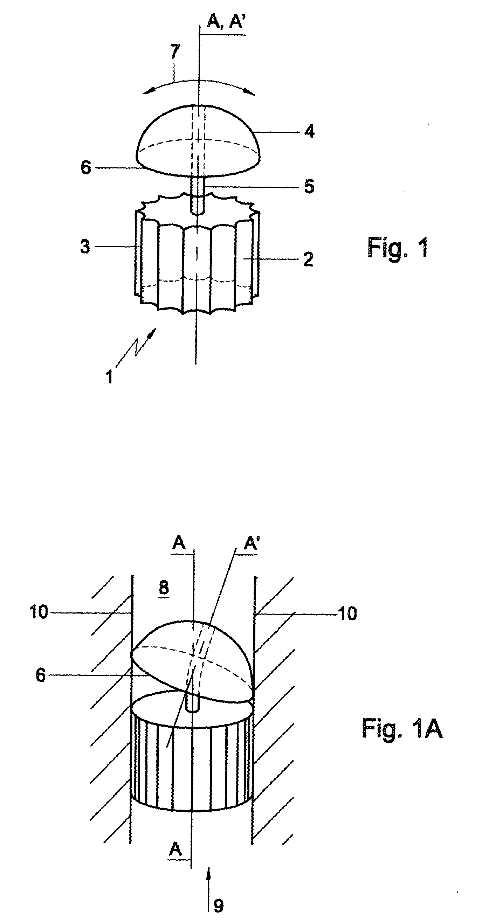

Figures 1A and 1B show the functional features of a plug 1 according to the invention. The plug 1 comprises a central body 2. The central body 2 is provided with fixing means 3 for fixing the plug 1 into a bone canal 8 by clamping engagement of the walls 10 of the bone canal. In the drawing, the fixing means 3 are shown as longitudinal ribs. The plug 1 further comprises separately configured blocking means 4 for blocking cement passage of the bone canal 8 by sealing engagement of the walls 10 of the bone canal. The fixing means 3 and the blocking means 4 extend radially outward relative to a central axis A of the plug. The blocking means 4 at the central body 2 are axially spaced, leaving room for swivelling movement of the blocking means 4 relative to the body 2. In the drawing, the blocking means are shown as a flexible dome.

The blocking means are swivably connected to the central body 2 by means of a swivable connection 5. In the drawing, the swivable connection 5 is shown as a flexible, cylindrical axle.

Upon insertion of the plug 1 into a bone canal, the blocking means 4 can swivel relative to the central body 2, such that a peripheral portion 6 of the blocking means 4 can sealingly engage with the walls of the bone canal. The swivelling movement is indicated with arrow 7.

To further elucidate operation of the plug 1, fig. 1B shows the plug 1 after it has been axially inserted into a bone canal 8 in the direction of arrow 9. The blocking means 4 have swivelled relative to the central body 2 to allow sealing engagement of the peripheral portion 6 of the blocking means 4 with the walls 10 of the bone canal 8. Cement passage through the bone canal is now blocked.

The fixing means 3 cooperate with the walls 10 of the bone canal 8, such that the plug 1 is axially fixed therein.

The swivelled movement of the blocking means is shown in fig. 1B by central axis A' of the blocking means 4, which in fig. 1A was coaxial with central axis A of the central body 2, and which axis A' now intersects central axis A.

It shall be clear that the degree of swivelling movement and the direction of swivelling movement may vary depending on the interaction between the blocking means 4 and the walls 10 of the bone canal 8.

The blocking means 4 and the fixing means 3 are configured separately to each fulfil their own function. However, it shall be clear that the blocking means 4 will to a degree also perform a fixing function and the fixing means 3 will also to a degree perform a blocking function.

It shall be clear from figs. 1A and 1B that the functional features of the plug 1 can be embodied in many ways. The blocking means can e.g. also be disk-shaped and the fixing means can e.g. also be embodied as radial ridges or studs. Furthermore, the swivable connection can e.g. also comprise a hinge or a bearing. The swivable connection may also be formed by a relatively stiff axis being mounted on a relatively flexible portion of the blocking means and/or the central body. The central body may be of different shape, e.g. ball-shaped.

It shall also be clear that the blocking means may also comprise several blocking elements and that the blocking means need not be centrally connected to the central body. Furthermore, the plug 1 may comprise further elements, such that the blocking means are interposed between the central body and the further elements.

It shall be clear that the swivable connection is preferably provided with sufficient stiffness to retain axial spacing between the blocking means and the central body during insertion. However, such axial spacing can be reinstated by retracting the plug slightly after insertion. It shall also be clear that the axial spacing need not be void but may be filled up with easily compressed (foam) material that allows tilting of the blocking means.

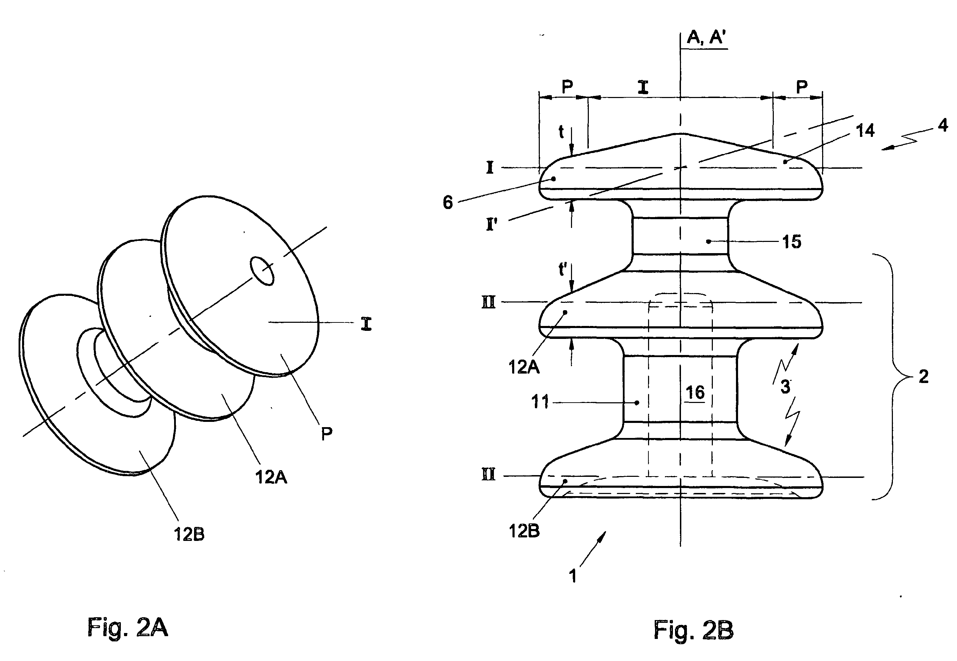

Figs. 2A and 2B show a currently preferred embodiment of the plug 1.

The plug 1 comprises a central body 2 carrying fixing means 3 for fixing the plug 1 in a bone canal 8 by clamping engagement of the walls of the bone canal. The plug 1 further comprises separately configured blocking means 4 for blocking cement passage through the bone canal by sealing engagement of the walls of the bone canal.

The central body 2 comprises a substantially cylindrical central axle 11 and the fixing means 3 comprise two radially outwardly extending flanges 12A and 12B carried on that axle 11. The flanges 12A, 12B have a substantially circular circumference. The flanges are substantially disk-shaped and are rounded-off at their peripheral portions 13A, 13B to facilitate insertion into the bone canal.

The blocking means 4 are embodied as a further flange 14. The further flange 14 is carried on a substantially cylindrical axial extension 15 of the central axis 11. The axial extension 15 forms a flexible axle portion by which the further flange 14 is axially spaced and swivably connected to the central body 2. Between the flange 12A and the further flange 14 a space exists to allow swivelling movement of the further flange 14 relative to the flange 12A.

The axial extension 15 has a reduced bending stiffness relative to the central axle 11. This is achieved by providing the axial extension 15 with a smaller diameter than the central axle 11. It shall be clear that the reduced relative bending stiffness can be achieved in many different ways. The axial extension 15 can e.g. also be made longer than the central axle 11, or can be slotted.

The thickness t of the further flange 14 is preferably chosen smaller than the thickness t' of the flanges 12A, 12B carried on the central portion to make it relatively flexible as a whole. Preferably, the blocking flange 14 comprises a relatively stiff inner portion I radially outwardly extending from axial extension 15 forming a central support to a relatively flexible peripheral portion P. Furthermore, the flanges 12 and/or the flanges 14 may be provided with a non-circular circumference, e.g. an oval circumference. Preferably, the flanges 12A, 12B and 14 have all substantially equal radial dimensions. In addition, the axial distance between the fixing flanges 12A and 12B can be chosen larger than the axial distance between the fixing flange 12B and the blocking flange 14, such that a relatively large free space exists between the fixing flanges 12A and 12B.

The central axle 11 may be provided with a chamber 16 in which the tip of a shaft can be inserted. The shaft can be used to insert the plug 1 into the bone canal 8. The chamber 16 with the tip of the shaft inserted therein will further increase relative bending stiffness of the central axle 11 compared to the axial extension 15. This way, during insertion, the flanges 12A, 12B will remain substantially parallel to each other and substantially perpendicular to the central axis of the bone canal, while the further flange 14 can swivel to a position wherein its plane I' intersects with the planes II through the flanges 12A, 12B and the peripheral portion 6 of the flange 14 can sealingly engage the walls 10 of the bone canal 8. The cylindrical shape of the axial extension allows it to bend relatively easily, such that the further flange can easily swivel to a position wherein a first peripheral portion 6A can move axially closer to the central body 2 while a radially opposite, peripheral portion 6B can move axially away from the central body 2 to extend deeper into the bone canal 8.

The plug 1 may be made of a biodegradable, swellable material and can be produced in one piece by injection molding.

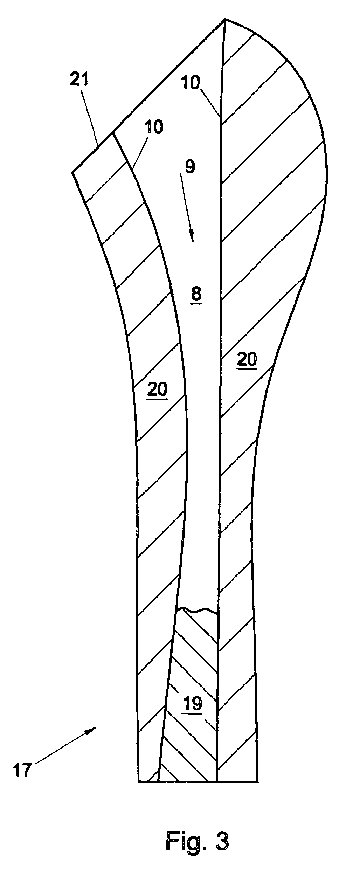

Figure 3 shows a femural bone 17 that has been prepared for implantation of a hip prothesis 18. A bone canal 8 is reamed by removing the porous trabecular bone 19, such that the hard, cortical bone 20 defines the walls 10 of the bone canal 8.

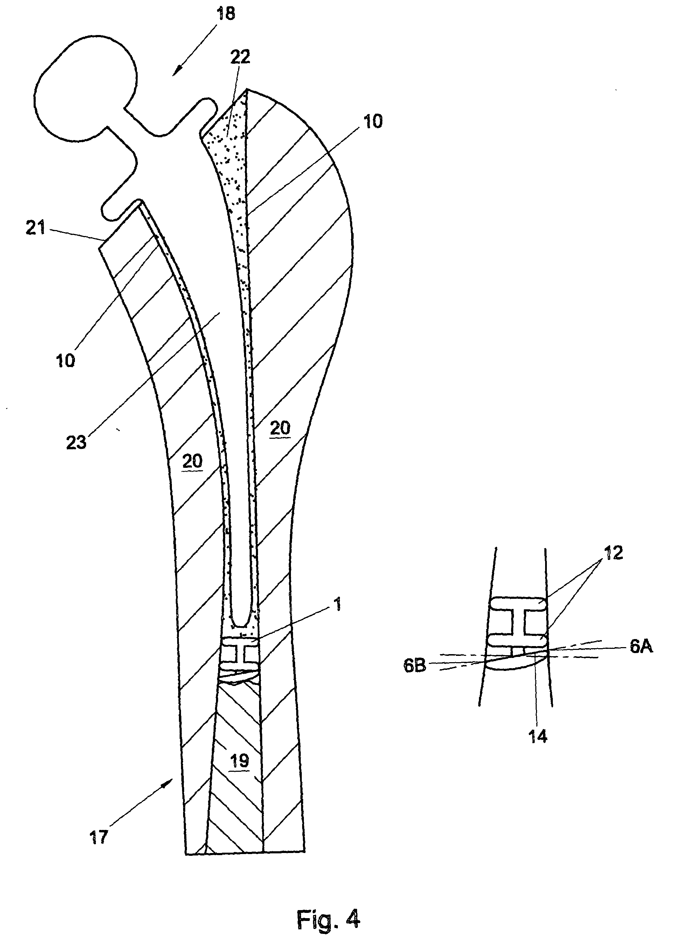

Referring to fig. 4, the plug 1 is inserted into the reamed bone canal 8. This may e.g. be carried out by inserting a shaft assembly carrying the plug 1 on its tip, by means of a screw thread connection to chamber 16 or other releasable connection, into the reamed bone canal 8. Upon insertion of the plug 1 by moving the shaft assembly in the direction of the arrow 9 inwards into the bone canal 8, the flanges 12A, 12B and 14 engage the walls 10 of the bone canal 8 and flex backwardly into a slide-shaped orientation. By providing a scale on the shaft assembly, the correct depth of insertion of the plug 1 into the canal 8 relative to the edge 21 of the femur 17 can be ensured. During insertion, the further flange 14 will swivel relative to the central body 2, such that its periferal portion 6 can sealingly follow the walls 10 of the bone canal. In the fully inserted position, shown in fig. 4, the shaft assembly is detached from the plug. The flanges 12A and 12B now fix the plug in the bone canal by clamping engagement, while the swivelled further flange 14 sealingly engages the walls of the bone canal and block cement passage thereof.

As is shown in fig. 4, subsequently cement 22 is inserted into the bone canal 8 and the prosthesis 10 is inserted into the bone canal such that its anchoring portion 23 is surrounded by cement 22 and no voids are formed in the cement.

It shall be clear to the skilled man that the plug is not limited to the preferred embodiments described herein and that many variations are possible within the scope of the appended claims.

In particular, it should be noted that the plugs can be used in bone canals in various bones of the body, e.g. in an upper arm bone canal for a shoulder prosthesis or a lower leg bone canal for a knee prosthesis.

## Claims

1. Plug for insertion into a bone canal, comprising a central body having fixing means for fixing the plug in the bone canal by clamping engagement of the walls of the bone canal, further comprising separate blocking means for blocking cement passage of the bone canal by sealing engagement of the walls of the bone canal, said fixing means and said blocking means extending radially outward relative to a central axis of the plug, in which plug the blocking means are axially spaced from said central body and are swivably connected thereto, such that, upon insertion of the plug into the bone canal, the blocking means can swivel relative to the central body to sealingly engage a peripheral portion of the blocking means with the walls of the bone canal.

2. Plug according to claim 1, wherein said blocking means have a substantially cylindrical circumference forming said peripheral portion.

3. Plug according to claim 1 or 2, wherein said blocking means comprise a substantially disk shaped element.

4. Plug according to any of the preceding claims, wherein the circumferential portion of said blocking means is made of relatively flexible material.

5. Plug according to any of the preceding claims, wherein the blocking means comprise a relatively stiff, inner portion radially outwardly extending from a central support to a relatively flexible, peripheral portion.

6. Plug according to any of the preceding claims, wherein the radial dimension of the blocking means is substantially equal to or greater than the radial dimension of the fixing means.

7. Plug according to any of the preceding claims, wherein the blocking means are swivably connected to said central body by means of a flexible axle portion.

8. Plug according to any of the preceding claims, wherein said central body comprises a central, substantially cylindrical axle and said fixing means comprise at least one radially outwardly extending flange carried on said axle.

9. Plug according to claim 8, wherein said fixing means comprise at least two circular flanges, carried axially spaced on said central axle.

10. Plug according to claim 8 or 9, wherein said blocking means comprise a further radially outwardly extending flange, carried on an axial extension of said central axle, the axial extension forming a substantially cylindrical flexible axle portion.

11. Plug according to claim 10, wherein said axial extension of said body has a reduced bending stiffness relative to the central axle.

12. Plug according to any of the preceding claims, wherein at least the fixing means and/or the blocking means comprise a swellable material.

13. Plug according to any of the preceding claims, made from a biocompatible and/or biodegradable material.

14. Method of blocking a bone canal, wherein a plug according to any of the preceding claims is inserted into a bone canal, such that blocking means of said plug are swivelled relative to a central body of the plug to sealingly engage a peripheral portion of the blocking means with the walls of the bone canal.
